# EUROPEAN PATENT APPLICATION

(11) **EP 4 371 513 A2**
(43) Date of publication of application: **22.05.2024**
(21) Application number: 24168292.1
(22) Date of filing: 09.08.2022
(51) Int. Cl.: A61B 18/12

(54) **SYSTEMS FOR ELECTROPORATION USING ASYMMETRIC WAVEFORMS AND WAVEFORMS WITH REDUCED BURST DURATION**

(30) Priority: 12.08.2021 US 202163232353 P; 07.12.2021 US 202163286711 P
(62) Divisional of application: 22189455.3
(71) Applicant: St. Jude Medical, Cardiology Division, Inc., St. Paul MN 55117 (US)
(72) Inventor: MARASS, Timothy S., Minneapolis, 55419 (US); TEGG, Troy T., Elk River, 55330 (US); DALY, Jacob J., Ham Lake, 55304 (US); SUTERMEISTER, Derek, Ham Lake, 55304 (US); MITTAL, Lakshya, Plymouth, (US); TRANTER, John, Minneapolis, 55417 (US); FISH, Jeffrey M., Maple Grove, 55311 (US)
(74) Representative: Alpspitz IP

(57) **Abstract**

Systems and methods for electroporation are provided. An electroporation system includes a catheter including a plurality of electrodes, and a pulse generator coupled to the catheter, the pulse generator configured to generate a waveform to be delivered using at least one of the plurality of electrodes. The waveform includes a first pulse having a first polarity, a first pulse amplitude, and a first pulse width, and a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.

## Description

### CROSS-REFERENCE TO RELATED APPLICATIONS

This application claims priority to provisional application serial No. 63/232,353, filed August 12, 2021, and provisional application serial No. 63/286,711, filed December 7, 2021, both of which are incorporated herein by reference in their entirety.

### FIELD OF THE DISCLOSURE

The present disclosure relates generally to tissue ablation systems. In particular, the present disclosure relates to applying electroporation therapy using waveforms with a reduced burst duration.

### BACKGROUND

It is generally known that ablation therapy may be used to treat various conditions afflicting the human anatomy. For example, ablation therapy may be used in the treatment of atrial arrhythmias. When tissue is ablated, or at least subjected to ablative energy generated by an ablation generator and delivered by an ablation catheter, lesions form in the tissue. Electrodes mounted on or in ablation catheters are used to cause tissue destruction in cardiac tissue to correct conditions such as atrial arrhythmia (including, but not limited to, ectopic atrial tachycardia, atrial fibrillation, and atrial flutter).

Arrhythmia (i.e., irregular heart rhythm) can create a variety of dangerous conditions including loss of synchronous atrioventricular contractions and stasis of blood flow which can lead to a variety of ailments and even death. It is believed that the primary cause of atrial arrhythmia is stray electrical signals within the left or right atrium of the heart. The ablation catheter imparts ablative energy (e.g., radiofrequency energy, cryoablation, lasers, chemicals, high-intensity focused ultrasound, etc.) to cardiac tissue to create a lesion in the cardiac tissue. This lesion disrupts undesirable electrical pathways and thereby limits or prevents stray electrical signals that lead to arrhythmias.

Electroporation is a non-thermal ablation technique that involves applying strong electric-fields that induce pore formation in the cellular membrane. The electric field may be induced by applying a relatively short duration pulse which may last, for instance, from a nanosecond to several milliseconds. Such a pulse may be repeated to form a pulse train. When such an electric field is applied to tissue in an in vivo setting, the cells in the tissue are subjected to an increased trans-membrane potential, which opens the pores on the cell plasma membrane. Electroporation may be reversible (i.e., the temporally-opened pores will reseal) or irreversible (i.e., the pores will remain open). For example, in the field of gene therapy, reversible electroporation (i.e., temporarily open pores) is used to transfect high molecular weight therapeutic vectors into the cells. In other therapeutic applications, a suitably configured pulse train alone may be used to cause cell destruction, for instance by causing irreversible electroporation.

For example, pulsed field ablation (PFA) may be used to perform instantaneous pulmonary vein isolation (PVI). PFA generally involves delivering high voltage pulses from electrodes disposed on a catheter. For example, voltage pulses may range from less than about 500 volts to about 2400 volts or higher. These fields may be applied between pairs of electrodes (bipolar therapy) or between one or more electrodes and a return patch (monopolar therapy).

In PFA, different waveforms may be used to achieve different goals. For example, some waveforms may result in larger or smaller lesion size than other waveforms. Further, some waveforms result in higher or lower overall energy delivery than other waveforms (less overall energy delivery generally corresponds to less heating of the target tissue). As another example, some waveforms are more likely to induce muscular contractions in a patient. Generally, it is desirable to deliver electroporation therapy with a relatively low number of therapy applications over a relatively short timeframe. Further, it is generally desirable to avoid thermal heating of the tissue, and to have little to no skeletal muscle recruitment (i.e., avoiding muscle contractions). In addition, it is also generally desirable to reduce the likelihood of waveforms generating sustained atrial arrhythmias.

### BRIEF SUMMARY OF THE DISCLOSURE

In one aspect, an electroporation system is provided. The electroporation system includes a catheter including a plurality of electrodes, and a pulse generator coupled to the catheter, the pulse generator configured to generate a waveform to be delivered using at least one of the plurality of electrodes. The waveform includes a first pulse having a first polarity, a first pulse amplitude, and a first pulse width, and a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.

In another aspect, a pulse generator for use with an electroporation system is provided. The pulse generator is configured to be coupled to a catheter including a plurality of electrodes and configured to generate a waveform to be delivered using at least one of the plurality of electrodes. The waveform includes a first pulse having a first polarity, a first pulse amplitude, and a first pulse width, and a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.

In yet another aspect, an electroporation system is provided. The electroporation system includes a catheter including a plurality of electrodes, and a pulse generator coupled to the catheter, the pulse generator configured to generate a waveform to be delivered using at least one of the plurality of electrodes. The waveform includes a plurality of windows, each window including a plurality of pulses, wherein the waveform has a burst duration that is less than approximately 176 milliseconds (ms) and a window period that is less than approximately 8ms.

The foregoing and other aspects, features, details, utilities, and advantages of the present disclosure will be apparent from reading the following description and claims, and from reviewing the accompanying drawings.

### ASPECTS

1. An electroporation system comprising:
   a catheter comprising a plurality of electrodes; and
   a pulse generator coupled to the catheter, the pulse generator configured to generate a waveform to be delivered using at least one of the plurality of electrodes, the waveform including:
      a first pulse having a first polarity, a first pulse amplitude, and a first pulse width; and
      a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.
2. The electroporation system in accordance with aspect 1, wherein the first polarity is opposite of the second polarity.
3. The electroporation system in accordance with aspect 1 or 2, wherein the first pulse amplitude is different than the second pulse amplitude, and wherein the first pulse width is different than the second pulse width.
4. The electroporation system in accordance with any one of the preceding aspects, wherein the pulse generator is configured to generate a waveform to be delivered to cardiac tissue.
5. The electroporation system in accordance with any one of the preceding aspects, wherein the waveform further includes a pulse pair including a third pulse and a fourth pulse, wherein the third pulse and the fourth pulse have the same pulse width and same pulse amplitude, and wherein the pulse pair either precedes the first pulse or follows the second pulse.
6. The electroporation system in accordance with any one of the preceding aspects, wherein the first pulse is a positive pulse.
7. The electroporation system in accordance with any one of the preceding aspects, wherein the second pulse is a positive pulse.
8. The electroporation system in accordance with any one of the preceding aspects, wherein the catheter is one of a loop catheter and a basket catheter.
9. The electroporation system in accordance with any one of the preceding aspects, wherein the catheter is configured to deliver the first and second pulses using a first pair of electrodes of the plurality of electrodes, wherein the waveform generator is further configured to generate a pulse pair including a third pulse and a fourth pulse, and wherein the catheter is configured to deliver the pulse pair using a second pair of electrodes of the plurality of electrodes, the second pair of electrodes different from the first pair of electrodes.
10. The electroporation system in accordance with any one of the preceding aspects, wherein the waveform includes a plurality of windows, each window including a plurality of pulses, wherein the waveform has a burst duration that is less than approximately 176 milliseconds (ms) and a window period that is less than approximately 8 milliseconds (ms).
11. A pulse generator for use with an electroporation system, the pulse generator configured to be coupled to a catheter including a plurality of electrodes and configured to generate a waveform to be delivered using at least one of the plurality of electrodes, the waveform including:
   a first pulse having a first polarity, a first pulse amplitude, and a first pulse width; and
   a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.
12. An electroporation system comprising:
   a catheter comprising a plurality of electrodes; and
   a pulse generator coupled to the catheter, the pulse generator configured to generate a waveform to be delivered using at least one of the plurality of electrodes, the waveform including:
      a plurality of windows, each window including a plurality of pulses, wherein the waveform has a burst duration that is less than approximately 176 milliseconds (ms) and a window period that is less than approximately 8ms.
13. The electroporation system in accordance with aspect 12, wherein the burst duration is less than 150ms.
14. The electroporation system in accordance with aspect 12 or 13, wherein the window period is less than 7ms.
15. The electroporation system in accordance with aspect 12, 13 or 14, wherein each window includes at least negative pulses.

### BRIEF DESCRIPTION OF THE DRAWINGS

Figure 1 is a schematic and block diagram view of a system for electroporation therapy.
Figures 2A and 2B are views of one embodiment of a catheter assembly that may be used with the system shown in Figure 1.
Figures 3A-3C are views of alternative embodiments of a catheter assembly that may be used with the system shown in Figure 1.
Figure 4 is one embodiment of a waveform that may be delivered using the system shown in Figure 1.
Figure 5 is an alternative waveform that may be delivered using the system shown in Figure 1.
Figure 6 is an alternative waveform that may be delivered using the system shown in Figure 1.
Figure 7 is an alternative waveform that may be delivered using the system shown in Figure 1.
Figure 8 is an embodiment of a window that is part of a larger monophasic waveform.
Figure 9 is an embodiment of a waveform including a plurality of windows.
Figure 10A is a schematic diagram of a catheter assembly that may be used to deliver the waveform shown in Figure 9.
Figure 10B is a chart of one embodiment of an electrode addressing scheme that may be used for the waveform shown in Figure 9.

### DETAILED DESCRIPTION OF THE DISCLOSURE

The present disclosure provides systems and methods for electroporation. An electroporation system includes a catheter including a plurality of electrodes, and a pulse generator coupled to the catheter, the pulse generator configured to generate a waveform to be delivered using at least one of the plurality of electrodes. The waveform includes a first pulse having a first polarity, a first pulse amplitude, and a first pulse width, and a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.

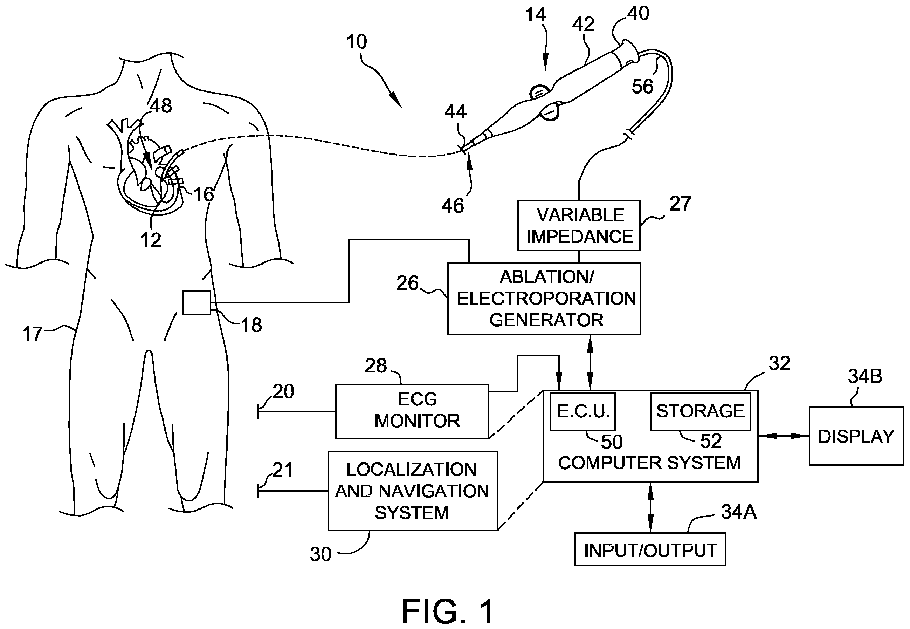

Figure 1 is a schematic and block diagram view of a system 10 for electroporation therapy. In general, system 10 includes a catheter electrode assembly 12 disposed at a distal end 48 of a catheter 14. As used herein, "proximal" refers to a direction toward the end of the catheter near the clinician and "distal" refers to a direction away from the clinician and (generally) inside the body of a patient. The electrode assembly includes one or more individual, electrically-isolated electrode elements. Each electrode element, also referred to herein as a catheter electrode, is individually wired such that it can be selectively paired or combined with any other electrode element to act as a bipolar or a multi-polar electrode.

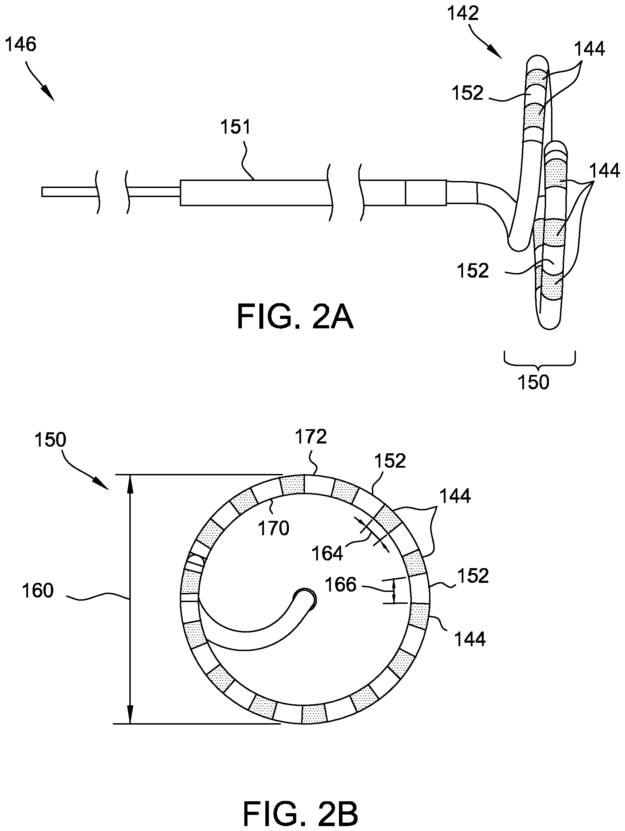

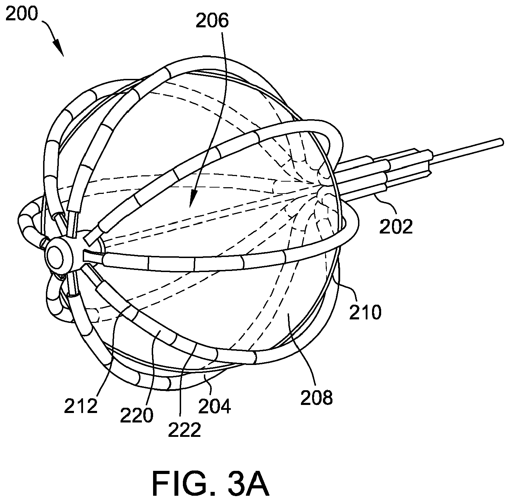

System 10 may be used for irreversible electroporation (IRE) to destroy tissue. In particular, system 10 may be used for electroporation-induced therapy that includes delivering electrical current in such a manner as to directly cause an irreversible loss of plasma membrane integrity leading to its breakdown and cell destruction. This mechanism of cell destruction may be viewed as an "outside-in" process, meaning that the disruption of the outside plasma membrane of the cell causes detrimental effects to the inside of the cell. Typically, for classical plasma membrane electroporation, electric current is delivered as a pulsed electric field in the form of short-duration pulses (e.g., having a 10 nanosecond (ns) to 100 millisecond (ms) duration) between closely spaced electrodes capable of delivering an electric field strength of about 0.1 to 3.0 kilovolts/centimeter (kV/cm). System 10 may be used for high output (e.g., high voltage and/or high current) electroporation procedures. Further, system 10 may be used with a loop catheter such as that depicted in Figures 2A and 2B, and/or with a basket catheter such as those depicted in Figures 3A-3C. In some embodiments, system 10 is used for reversible electroporation instead of or in addition to irreversible electroporation.

In one embodiment, stimulation is delivered selectively (e.g., between pairs of electrodes) on catheter 14. Further, the electrodes on catheter 14 may be switchable between being connected to a 3D mapping system and being connected to an electroporation generator.

Irreversible electroporation through a multi-electrode catheter may enable pulmonary vein isolation in as few as one shock per vein, which may produce much shorter procedure times compared to sequentially positioning a radiofrequency (RF) ablation tip around a vein.

It should be understood that while the energization strategies are described as involving square wave pulses, embodiments may use variations and remain within the spirit and scope of the disclosure. For example, exponentially-decaying pulses, exponentially-increasing pulses, and combinations may be used.

Further, it should be understood that the mechanism of cell destruction in electroporation is not primarily due to heating effects, but rather to cell membrane disruption through application of a high-voltage electric field. Thus, electroporation may avoid some possible thermal effects that may occur when using radio frequency (RF) energy. This "cold therapy" thus has desirable characteristics.

With this background, and now referring again to Figure 1, system 10 includes a catheter electrode assembly 12 including at least one catheter electrode. Electrode assembly 12 is incorporated as part of a medical device such as a catheter 14 for electroporation therapy of tissue 16 in a body 17 of a patient. In the illustrative embodiment, tissue 16 includes heart or cardiac tissue. It should be understood, however, that embodiments may be used to conduct electroporation therapy with respect to a variety of other body tissues (e.g., renal tissue, tumors, etc.).

Figure 1 further shows a plurality of return electrodes designated 18, 20, and 21, which are diagrammatic of the body connections that may be used by the various sub-systems included in overall system 10, such as an electroporation generator 26, an electrophysiology (EP) monitor such as an ECG monitor 28, and a localization and navigation system 30 for visualization, mapping, and navigation of internal body structures. In the illustrated embodiment, return electrodes 18, 20, and 21 are patch electrodes. It should be understood that the illustration of a single patch electrode is diagrammatic only (for clarity) and that such sub-systems to which these patch electrodes are connected may, and typically will, include more than one patch (body surface) electrode, and may include split patch electrodes (as described herein). In other embodiments, return electrodes 18, 20, and 21 may be any other type of electrode suitable for use as a return electrode including, for example, one or more catheter electrodes. Return electrodes that are catheter electrodes may be part of electrode assembly 12 or part of a separate catheter or device (not shown). System 10 may further include a main computer system 32 (including an electronic control unit 50 and data storage-memory 52), which may be integrated with localization and navigation system 30 in certain embodiments. System 32 may further include conventional interface components, such as various user input/output mechanisms 34A and a display 34B, among other components.

Electroporation generator 26 is configured to energize the electrode element(s) in accordance with an electroporation energization strategy, which may be predetermined or may be user-selectable. For electroporation therapy, generator 26 may be configured to produce an electric current that is delivered via electrode assembly 12 as a pulsed electric field in the form of short-duration square wave pulses (e.g., a nanosecond to several milliseconds duration, or any duration suitable for electroporation) between closely spaced electrodes capable of delivering an electric field strength (i.e., at the tissue site) of about 0.1 to 3.0 kV/cm. The amplitude and pulse width needed for irreversible electroporation are inversely related. That is, as pulse widths are decreased, the amplitude may generally be increased to achieve chronaxie.

Electroporation generator 26, sometimes also referred to herein as a DC energy source, is a biphasic electroporation generator 26 configured to generate a series of energy pulses that all produce current in two directions (i.e., positive and negative pulses). In other embodiments, electroporation generator is a monophasic or polyphasic electroporation generator. In some embodiments, electroporation generator 26 is configured to output energy in pulses at selectable energy levels, such as fifty joules, one hundred joules, two hundred joules, and the like. Other embodiments may have more or fewer energy settings and the values of the available setting may be the same or different. For successful electroporation, some embodiments utilize the two hundred joule output level. For example, electroporation generator 26 may output a pulse having a peak magnitude from about 300 Volts (V) to about 3,200 V. Other embodiments may output any other suitable positive or negative voltage.

In some embodiments, a variable impedance 27 allows the impedance of system 10 to be varied to limit arcing. Moreover, variable impedance 27 may be used to change one or more characteristics, such as amplitude, duration, pulse shape, and the like, of an output of electroporation generator 26. Although illustrated as a separate component, variable impedance 27 may be incorporated in catheter 14 or generator 26.

With continued reference to Figure 1, as noted above, catheter 14 may include functionality for electroporation and in certain embodiments also additional ablation functions (e.g., RF ablation). It should be understood, however, that in those embodiments, variations are possible as to the type of ablation energy provided (e.g., cryoablation, ultrasound, etc.).

In the illustrative embodiment, catheter 14 includes a cable connector or interface 40, a handle 42, and a shaft 44 having a proximal end 46 and a distal 48 end. Catheter 14 may also include other conventional components not illustrated herein such as a temperature sensor, additional electrodes, and corresponding conductors or leads. Connector 40 provides mechanical and electrical connection(s) for cable 56 extending from generator 26. Connector 40 may include conventional components known in the art and as shown is disposed at the proximal end of catheter 14.

Handle 42 provides a location for the clinician to hold catheter 14 and may further provide means for steering or the guiding shaft 44 within body 17. For example, handle 42 may include means to change the length of a guidewire extending through catheter 14 to distal end 48 of shaft 44 or means to steer shaft 44. Moreover, in some embodiments, handle 42 may be configured to vary the shape, size, and/or orientation of a portion of the catheter, and it will be understood that the construction of handle 42 may vary. In an alternate embodiment, catheter 14 may be robotically driven or controlled. Accordingly, rather than a clinician manipulating a handle to advance/retract and/or steer or guide catheter 14 (and shaft 44 thereof in particular), a robot is used to manipulate catheter 14. Shaft 44 is an elongated, tubular, flexible member configured for movement within body 17. Shaft 44 is configured to support electrode assembly 12 as well as contain associated conductors, and possibly additional electronics used for signal processing or conditioning. Shaft 44 may also permit transport, delivery and/or removal of fluids (including irrigation fluids and bodily fluids), medicines, biologics, and/or surgical tools or instruments. Shaft 44 may be made from conventional materials such as polyurethane and defines one or more lumens configured to house and/or transport electrical conductors, fluids or surgical tools, as described herein. Shaft 44 may be introduced into a blood vessel or other structure within body 17 through a conventional introducer. Shaft 44 may then be advanced/retracted and/or steered or guided through body 17 to a desired location such as the site of tissue 16, including through the use of guidewires or other means known in the art.

Localization and navigation system 30 may be provided for visualization, mapping and navigation of internal body structures. Localization and navigation system 30 may include conventional apparatus known generally in the art. For example, localization and navigation system 30 may be substantially similar to the EnSite Precision^{™} System, commercially available from Abbott Laboratories, and as generally shown in commonly assigned U.S. Pat. No. 7,263,397 titled "Method and Apparatus for Catheter Navigation and Location and Mapping in the Heart", the entire disclosure of which is incorporated herein by reference. In another example, localization and navigation system 30 may be substantially similar to the EnSite X^{™} System, as generally shown in U.S. Pat. App. Pub. No. 2020/0138334 titled "Method for Medical Device Localization Based on Magnetic and Impedance Sensors", the entire disclosure of which is incorporated herein by reference. It should be understood, however, that localization and navigation system 30 is an example only, and is not limiting in nature. Other technologies for locating/navigating a catheter in space (and for visualization) are known, including for example, the CARTO navigation and location system of Biosense Webster, Inc., the Rhythmia^{®} system of Boston Scientific Scimed, Inc., the KODEX^{®} system of Koninklijke Philips N.V., the AURORA^{®} system of Northern Digital Inc., commonly available fluoroscopy systems, or a magnetic location system such as the gMPS system from Mediguide Ltd.

In this regard, some of the localization, navigation and/or visualization systems may include a sensor for producing signals indicative of catheter location information, and may include, for example, one or more electrodes in the case of an impedance-based localization system, or alternatively, one or more coils (i.e., wire windings) configured to detect one or more characteristics of a magnetic field, for example in the case of a magnetic-field based localization system. As yet another example, system 10 may utilize a combination electric field-based and magnetic field-based system as generally shown with reference to U.S. Pat. No. 7,536,218 entitled "Hybrid Magnetic-Based and Impedance Based Position Sensing," the disclosure of which is incorporated herein by reference in its entirety.

Pulsed field ablation (PFA), which is a methodology for achieving irreversible electroporation, may be implemented using the systems and methods described herein. In some cases, PFA may be used at specific cardiac tissue sites such as the pulmonary veins to perform a pulmonary vein isolation (PVI). In PFA, electric fields may be applied between adjacent electrodes (in a bipolar approach) or between one or more electrodes and a return patch (in a monopolar approach). There are advantages and disadvantages to each of these approaches.

For lesion size and proximity, the monopolar approach has a wider range of effect, and can potentially create deeper lesions with the same applied voltage. Further, the monopolar approach may be able to create lesions from a distance (e.g., generally proximate, but not necessarily contacting tissue). The bipolar approach may create smaller lesions, requiring closer proximity or contact with tissue to create transmural lesions. However, the monopolar approach may create larger lesions than are necessary, while the lesions generated using the bipolar approach may be more localized.

Due to a wider range of effect, the monopolar approach may cause unwanted skeletal muscle and/or nerve activation. In contrast, the bipolar approach has a constrained range of effect proportional to electrode spacing on the lead, and is less likely to depolarize cardiac myocytes or nerve fibers.

To monitor operation of system 10, one or more impedances between catheter electrodes 144 and/or return electrodes 18, 20, and 21 may be measured. For example, for system 10, impedances may be measured as described in U.S. Patent Application Publication No. 2019/0117113, filed on October 23, 2018, U.S. Patent Application Publication No. 2019/0183378, filed on December 19, 2018, and U.S. Patent Application No. 63/027,660, filed on May 20, 2020, all of which are incorporated by reference herein in their entirety.

Figures 2A and 2B are views of one embodiment of a catheter assembly 146 that may be used with catheter 14 in system 10. Catheter assembly 146 may be referred to as a loop catheter. Those of skill in the art will appreciate that, in other embodiments, any suitable catheter may be used. Specifically, Figure 2A is a side view of catheter assembly 146 with a variable diameter loop 150 at a distal end 142. Figure 2B is an end view of variable diameter loop 150 of catheter assembly 146. Those of skill in the art will appreciate that the methods and systems described herein may be implemented using any suitable catheter (e.g., fixed loop catheters, linear catheters, basket catheter, etc.). As shown in Figures 2A and 2B, variable diameter loop 150 is coupled to a distal section 151 of shaft 44.

Variable diameter loop 150 is selectively transitionable between an expanded (also referred to as "open") diameter 160 (shown in Figure 2A) and a retracted (also referred to as "closed") diameter 160 (not shown). In the example embodiment, an expanded diameter 160 is twenty eight mm and a retracted diameter 160 is fifteen mm. In other embodiments, diameter 160 may be variable between any suitable open and closed diameters 160.

In the embodiment shown, variable diameter loop 150 includes fourteen catheter electrodes 144 substantially evenly spaced around the circumference of variable diameter loop 150 in the expanded configuration. In the retracted configuration, one or more of electrodes 144 may overlap. In other embodiments, other arrangements of catheter electrodes 144 may be implemented. For example, in one embodiment, variable diameter 1oop 150 includes twelve catheter electrodes 144.

Catheter electrodes 144 are platinum ring electrodes configured to conduct and/or discharge electrical current in the range of one thousand volts and/or ten amperes. In other embodiments, variable diameter loop 150 may include any suitable number of catheter electrodes 144 made of any suitable material. Catheter electrodes 144 may include any catheter electrode suitable to conduct high voltage and/or high current (e.g., in the range of one thousand volts and/or ten amperes). Each catheter electrode 144 is separated from each other catheter electrode by an insulated gap 152. In the example embodiment, each catheter electrode 144 has a same length 164 (shown in Figure 2B) and each insulated gap 152 has a same length 166 as each other gap 152. Length 164 and length 166 are both about 2.5 mm in the example embodiment. In other embodiments, length 164 and length 166 may be different from each other. Moreover, in some embodiments, catheter electrodes 144 may not all have the same length 164 and/or insulated gaps 152 may not all have the same length 166. In some embodiments, catheter electrodes 144 are not spaced evenly around the circumference of variable diameter loop 150.

Diameter 160 and catheter electrode 144 spacing may be developed to provide a targeted range of energy density to tissue, as well as to provide sufficient electroporation coverage for different human anatomic geometries. In general, a sufficient number of electrodes 144 with appropriate lengths 164 are desired to provide substantially even and continuous coverage around the circumference of variable diameter loop 150, while still allowing enough flexibility to allow variable diameter loop 150 to expand and contract to vary diameter 160 to the desired extremes.

As mentioned above, length 164 of catheter electrodes 144 may be varied. Increasing length 164 of catheter electrodes 144 may increase coverage of electrodes 144 around the circumference of variable diameter loop 150 while also decreasing current density (by increasing the surface area) on electrodes 144, which may help prevent arcing during electroporation operations. Increasing length 164 too much, however, may prevent variable diameter loop 150 from forming a smooth circular shape and may limit the closed diameter 160 of variable diameter loop 150. Additionally, too great a length 164 may increase the surface area of catheter electrodes 144 to a point that the current density applied to catheter electrodes 144 by a power source is below the minimum current density needed for successful therapy. Conversely, decreasing length 164 decreases the surface area, thereby increasing the current density (assuming no other system changes) on catheter electrodes 144. As discussed above, greater current densities may lead to increased risk of arcing during electroporation, and may result in larger additional system resistances needing to be added to prevent arcing. Moreover, in order to get a desired, even coverage about the circumference of variable diameter loop 150, more catheter electrodes 144 may be needed if length 164 is decreased. Increasing the number of catheter electrodes 144 on variable diameter loop 150 may prevent variable diameter loop 150 from being able to be contracted to a desired minimum diameter 160.

Figure 3A is a perspective view of an alternative catheter assembly 200 that may be used with catheter 14. Catheter assembly 200 may be referred to as a basket catheter. Catheter assembly 200 includes a shaft 202 and a plurality of splines 204 surrounding a distal portion 206 of shaft 202. In this embodiment, catheter assembly 200 also includes a balloon 208 enclosed by splines 204. Balloon 208 may be selectively inflated to fill the space between splines 204. Notably, balloon 208 functions as an insulator, and generally reduces energy losses, which may result in increased lesion size.

Each spline 204 includes a proximal end 210 coupled to shaft 202 and a distal end 212 coupled to shaft 202. From proximal end 210 to distal end 212, spline 204 has an arcuate shape that extends radially outward.

In this embodiment, each spline 204 includes a plurality of individual electrodes 220. For example, each spline 204 may include an elastic material (e.g., Nitinol) covered in a polymer tube 222, with individual electrodes 220 attached to an exterior of polymer tube 222. In the embodiment shown, each spline 204 includes two electrodes 220. Further, as shown in Figure 2, electrodes 220 are generally positioned closer to distal end 212 than proximal end 210 to correspond to portions of spline 204 that will contact the pulmonary vein.

Alternatively, each spline 204 may include any suitable number and arrangement of electrodes 220. For example, in some embodiments, each spline 204 includes four electrodes 220.

In this embodiment, alternating splines 204 alternate polarities. That is, electrodes 220 on a particular spline 204 have the same polarity, but electrodes 220 on a particular spline 204 have a different polarity than electrodes 220 on adjacent splines 204. Alternatively, any suitable polarization scheme may be used. During delivery, splines 204 may be collapsed in towards shaft 202. Subsequently, to perform ablation, splines 204 are deployed to extend radially outward.

Splines 204 may all have the same length, or at least some of splines 204 may have different lengths. Further, insulating material on each spline 204 may have the same length, or at least some splines 204 may have insulating material with different lengths. In addition, in some embodiments, catheter assembly 200 includes a distal electrode (not shown) positioned distal of splines 204. The distal electrode may be used to perform point ablation (e.g., by creating a bipole between the distal electrode and one of splines 204), and/or may be used for visualization/mapping purposes (e.g., using the distal electrode in combination with an electrode on shaft 202).

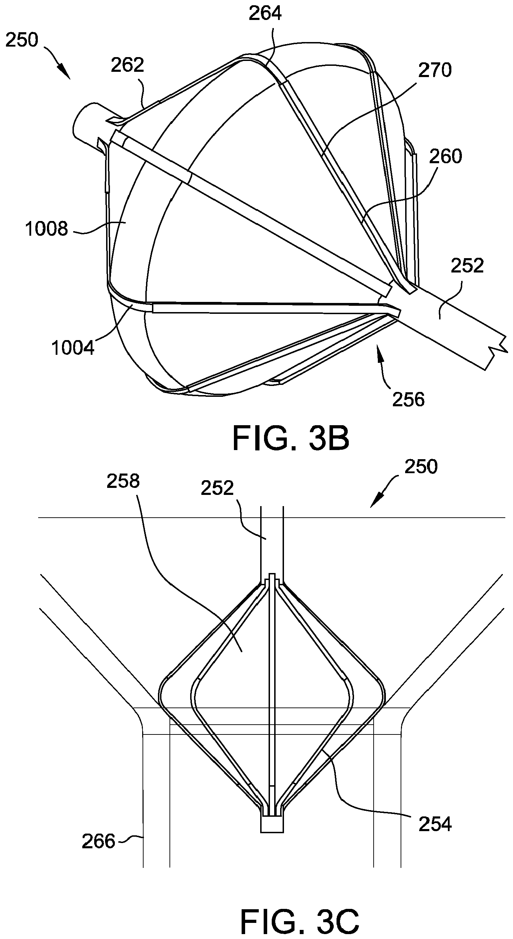

Figure 3B is a perspective view of an alternative catheter assembly 250 that may be used with catheter 14, and Figure 3C is a side schematic view of catheter assembly 250. Like catheter assembly 200 (shown in Figure 3A), catheter assembly 250 may be referred to as a basket assembly.

Catheter assembly 250 includes a shaft 252 and a plurality of splines 254 surrounding a distal portion 256 of shaft 252. In this embodiment, catheter assembly 250 includes a balloon 258 enclosed by splines 254. Balloon 258 may be selectively inflated to occupy the space between splines 254. Notably, balloon 258 functions as an insulator, and generally reduces energy, which may result in increased lesion size.

Each spline 254 includes a proximal end 260 coupled to shaft 252 and a distal end 262 coupled to shaft 252. From proximal end 260, spline 1004 extends radially outward to an inflection point 264, and then extends radially inward to distal end 262. Figure 3C shows catheter assembly 250 positioned within the pulmonary vein 266.

A body of each spline 254 is made of an elastic material (e.g., Nitinol), and functions as a relatively large electrode. In this embodiment, alternating splines 254 alternate polarities. That is, each positive spline 254 is positioned between two negative splines 254 and vice-versa. Alternatively, any suitable polarization scheme may be used.

To control the ablation zone of each spline 254 , portions of each spline 254 may be covered with insulating material 270 (e.g., heat-shrink or polymer tubing or spray or dip coat with polyimide or PEBAX), and the exposed portions of splines 254 function as electrodes. In the embodiment shown in Figures 3B and 3C, inflection point 264 and portions of spline 254 between inflection point 264 and distal end 262 are generally exposed, while portions of spline 254 between inflection point 264 and proximal end 260 are generally insulated. This results in the portions of spline 254 that contact pulmonary vein 266 being exposed (see Figure 3C). Alternatively, any suitable insulation configuration may be used.

During delivery, splines 254 and balloon 258 may be collapsed. To perform ablation, splines 254 are deployed with inflection points 264 extending radially outward, and balloon 258 is selectively inflated to occupy the space between splines 254.

The combination of balloon 258 and splines 254 facilitates straightforward delivery and deployment of catheter assembly 250. Further, balloon 258 drives more energy into ablated tissue, and stabilizes splines 254 to prevent lateral movement. In addition, using splines 254 as electrodes instead of individual smaller electrodes may facilitate reducing the cost and increasing the reliability of catheter assembly 250.

Splines 254 may all have the same length, or at least some of splines 254 may have different lengths. Further, insulating material 270 on each spline 254 may have the same length, or at least some splines 254 may have insulating material 270 with different lengths. In addition, in some embodiments, catheter assembly 250 includes a distal electrode (not shown) positioned distal of splines 254. The distal electrode may be used to perform point ablation (e.g., by creating a bipole between the distal electrode and one of splines 254), and/or may be used for visualization/mapping purposes (e.g., using the distal electrode in combination with an electrode on shaft 252).

Those of skill the art will appreciate that catheter assembly 146 (shown in Figure 2A and 2B), catheter assembly 200 (shown in Figure 3A), and catheter assembly 250 (shown in Figures 3B and 3C) are merely examples. Notably, the systems and methods described herein may be implemented using any suitable catheter assembly.

For electroporation therapy, waveforms are generated using a pulse generator (e.g., electroporation generator 26 (shown in Figure 1)) and applied between pairs of catheter electrodes (i.e., a bipolar approach) or between individual catheter electrodes and a return patch (i.e., a monopolar approach). The waveforms may be monophasic, biphasic (i.e., having both a positive pulse and a negative pulse), or polyphasic. Further, the waveforms may include one or more bursts of pulses (with each burst including multiple pulses). Further, the waveforms are defined by multiple parameters (e.g., pulse width, pulse amplitude, frequency, etc.).

Different waveforms may be used to achieve different goals. For example, some waveforms may result in larger or smaller lesion size than other waveforms. Further, some waveforms result or higher or lower overall energy delivery than other waveforms (less overall energy delivery generally corresponds to less heating of the target tissue). As another example, some waveforms are more likely to induce muscular contractions in a patient. Generally, it is desirable to deliver electroporation therapy with a relatively low number of therapy applications over a relatively short timeframe. Further, it is generally desirable to avoid thermal heating of the tissue, and to have little to no skeletal muscle recruitment (i.e., avoiding muscle contractions).

The systems and method described herein use asymmetric waveforms for electroporation therapy. As discussed below, these asymmetric waveforms generally include a first pulse, a second pulse, and an interpulse delay between the first and second pulses. Further, the first and second pulses have a different amplitude and/or a different pulse width from one another (i.e., the first and second pulses are asymmetric). The first and second pulses may have opposite polarity (i.e., a positive pulse followed by a negative pulse, or a negative pulse followed by a positive pulse), or may have the same polarity (i.e., two positive pulses, or two negative pulses).

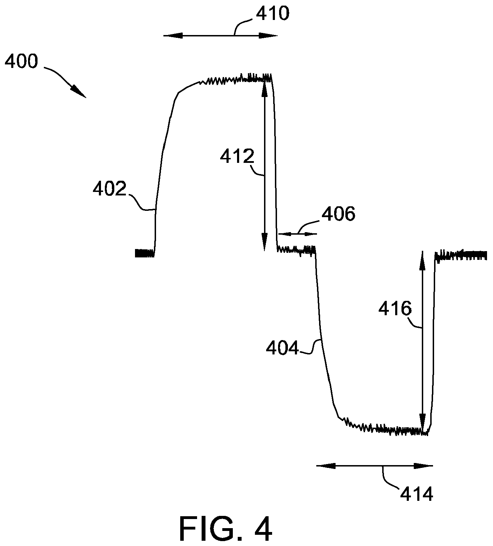

Figure 4 is one embodiment of a waveform 400 including a symmetric pulse pair. Waveform 400 includes a positive pulse 402 followed by a negative pulse 404. Further, there is an interpulse delay 406 between the positive and negative pulses 402 and 404.

As shown in Figure 4, positive pulse 402 has a first pulse width 410 and a first pulse amplitude 412. Similarly, negative pulse 404 has a second pulse width 414 and a second pulse amplitude 416. Waveform 400 includes a symmetric pulse pair. Accordingly, first pulse width 410 is substantially equal to second pulse width 414, and first pulse amplitude 412 is substantially equal to second pulse amplitude 416.

For example, first and second pulse widths 410 and 414 may each be 3 microseconds (3µs), with an interpulse delay of 1µs. This may be referred to as a 3-1-3 waveform (i.e., first pulse width of 3µs - interpulse delay of 1µs - second pulse width of 3µs). First and second pulse amplitudes 412 and 416 may each be, for example, on the order of 1800 Volts (1800V).

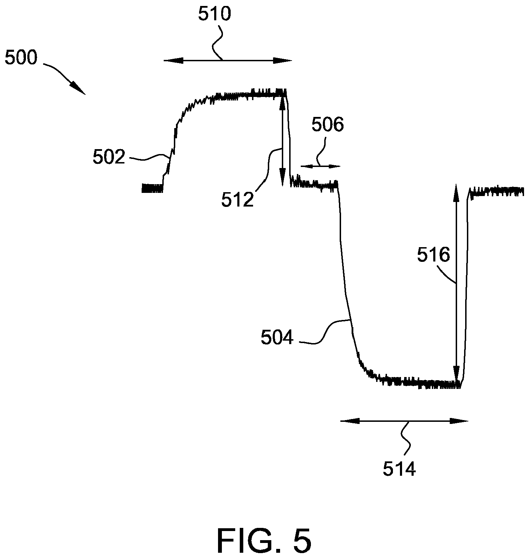

Figure 5 is one embodiment of a waveform 500 including an asymmetric pulse pair. Waveform 500 includes a positive pulse 502 followed by a negative pulse 504. Those of skill in the art will appreciate that waveform 500 may alternatively include negative pulse 504 followed by positive pulse 502 (or two negative pulses, or two positive pulses). Further, there is an interpulse delay 506 between the positive and negative pulses 502 and 504.

As shown in Figure 5, positive pulse 502 has a first pulse width 510 and a first pulse amplitude 512. Similarly, negative pulse 504 has a second pulse width 514 and a second pulse amplitude 516. First pulse width 510 is substantially equal to second pulse width 514. However, first pulse amplitude 512 is different than second pulse amplitude 516, resulting in an asymmetric pulse pair.

For example, first and second pulse widths 510 and 514 may each be 3µs, with an interpulse delay of 1µs (i.e., a 3-1-3 waveform). However, first pulse amplitude 512 may be 600V, and second pulse amplitude 516 may be 1800V. Those of skill in the art will appreciate that these are example values, and any suitable pulse with and pulse amplitude values may be implemented. Waveforms similar to waveform 500 may result in reduced overall energy delivery and larger lesions than symmetric pulse pairs.

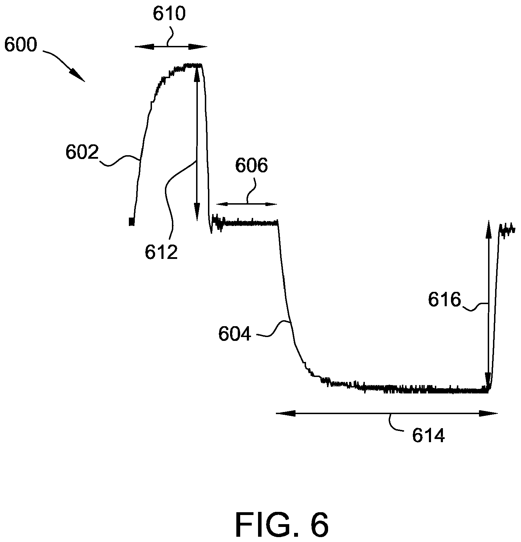

Figure 6 is another embodiment of a waveform 600 including an asymmetric pulse pair. Waveform 600 includes a positive pulse 602 followed by a negative pulse 604. Those of skill in the art will appreciate that waveform 600 may alternatively include negative pulse 604 followed by positive pulse 602 (or two negative pulses, or two positive pulses). Further, there is an interpulse delay 606 between the positive and negative pulses 602 and 604.

As shown in Figure 6, positive pulse 602 has a first pulse width 610 and a first pulse amplitude 612. Similarly, negative pulse 604 has a second pulse width 614 and a second pulse amplitude 616. First pulse amplitude 612 is substantially equal to second pulse amplitude 616. However, first pulse width 610 is different than second pulse width 614, resulting in an asymmetric pulse pair.

For example, first and second pulse amplitudes 612 and 616 may each be 1800V. However, first pulse width 610 may be 1µs, second pulse width 614 may be 3µs, and interpulse delay 606 may be 1µs (i.e., a 1-1-3 waveform). Those of skill in the art will appreciate that these are example values, and any suitable pulse with and pulse amplitude values may be implemented. Waveforms similar to waveform 600 may allow for a higher voltage level, larger lesion size, and reduced number of therapy applications relative to symmetric pulse pairs.

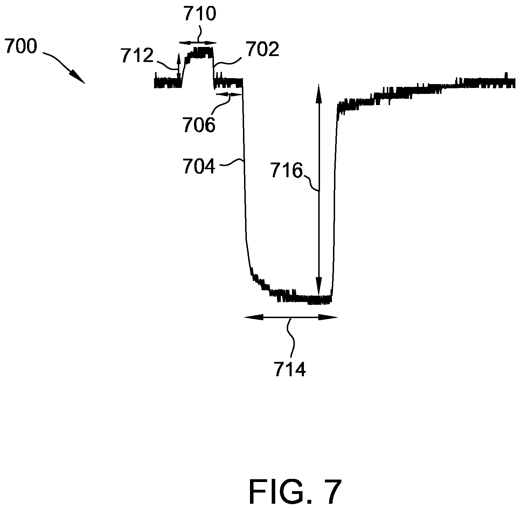

Figure 7 is another embodiment of a waveform 700 including asymmetric pulse pairs. Waveform 700 includes a positive pulse 702 followed by a negative pulse 704. Those of skill in the art will appreciate that waveform 700 may alternatively include negative pulse 704 followed by positive pulse 702 (or two negative pulses, or two positive pulses). Further, there is an interpulse delay 706 between the positive and negative pulses 702 and 704.

As shown in Figure 7, positive pulse 702 has a first pulse width 710 and a first pulse amplitude 712. Similarly, negative pulse 704 has a second pulse width 714 and a second pulse amplitude 716. In this embodiment, first pulse amplitude 712 is different than second pulse amplitude 716, and first pulse width 710 is different than second pulse width 714, resulting in an asymmetric pulse pair.

For example, first pulse amplitude 712 may be 300V, and second pulse amplitude 716 may be 1800V. Further, first pulse width 710 may be 1µs, second pulse width 714 may be 3µs, and interpulse delay 706 may be 1µs (i.e., a 1-1-3 waveform). Those of skill in the art will appreciate that these are example values, and any suitable pulse with and pulse amplitude values may be implemented.

Waveforms similar to waveform 700 may allow for a higher voltage level, larger lesion size, and reduced number of therapy applications relative to symmetric pulse pairs. Further waveforms similar to waveform 700 may result in reduced overall energy delivery and larger lesions than symmetric pulse pairs.

In some embodiments, a waveform can include pulses all having the same polarity (e.g., all positive pulses or all negative pulses). Delivering pulses all having the same polarity may result in larger lesions, and lower overall energy delivery (reducing tissue heating). However, delivering pulses all having the same polarity may also result in higher levels of skeletal muscle recruitment. Accordingly, to resolve this, a hybrid waveform including several pulses of one polarity and only a few pulses of the opposite polarity may be used. For example, a hybrid waveform may include a single positive pulse, followed by a plurality of negative pulses, followed by another single positive pulse.

As another example, in some embodiments, a waveform includes only a single pulse of a single polarity (e.g., a 0-0-3 waveform). This may be referred to as a monophasic waveform. This waveform may repeat multiple times as part of a larger waveform.

Notably, the pulse pairs of Figures 4-7 may be part of a larger waveform. For example, a burst of pulses may include a sequential series of multiple pulse pairs.

In one embodiment, a burst begins with a relatively mild waveform (e.g., a 1-1-1 waveform), followed by a more aggressive waveform (e.g., a 1-1-3 waveform), followed by another relatively mild waveform. The pulse amplitudes within each pulse pair may be symmetric or asymmetric. Further, those of skill in the art will appreciate that any suitable combination of parameters may be used to improve lesion size, reduce heating, and/or reduce skeletal muscle recruitment.

In another embodiment, a burst includes alternating pulse pairs. For example, a burst may begin with a relatively mild waveform (e.g., a 1-1-1 waveform) and end with a subsequent, more aggressive waveform (e.g., a 1-1-3 waveform).

Further, in embodiments implemented using a multiplexing delivery scheme, a first pair of electrodes may deliver a relatively mild waveform (e.g., a 1-1-1 waveform), and a second pair of electrodes may deliver a more aggressive waveform (e.g., a 1-1-3 waveform). The two waveforms may be delivered by the different pairs of electrodes sequentially or simultaneously. As another example, in some embodiments, a waveform delivered from one pair of electrodes may be temporally nested between waveforms delivered by another pair of electrodes.

In yet another embodiment, an overall pulse period may include a symmetric arrangement of asymmetric pulse pairs. For example, the overall pulse period may include two 1-1-3 waveforms, followed by two 3-1-1 waveforms, followed by two more 1-1-3 waveforms.

Again, in any of the embodiments described herein, any suitable combination of parameters may be used to improve lesion size, reduce overall energy delivery, reduce heating, and/or reduce skeletal muscle recruitment. For example, a 1-1-3 waveform requires two-thirds of the energy of an otherwise equivalent 3-1-3 waveform. Further, in general, to reduce tissue heating, pulse amplitude may be decreased while increasing the number of pulses. As an example, pulse amplitudes in the waveforms described herein may range from less than 1200V to more than 2400V. Further, pulse widths in the waveforms described herein may range from less than 1µs to more than 5µs.

As discussed above, waveforms for use in electroporation may be biphasic and symmetric (e.g., as shown in Figure 4), biphasic and asymmetric (e.g., as shown in Figures 5-7), or monophasic (i.e., including pulses all having the same polarity). In some embodiments, relative to biphasic waveforms, monophasic waveforms may facilitate increasing an ablation area, reducing thermal damage, and reducing overall therapy time. However, in some embodiments, monophasic waveforms may increase the likelihood of generation of sustained atrial arrhythmias relative to biphasic waveforms, which is generally undesirable. Notably, by reducing a burst duration of a monophasic waveform, the likelihood of generation of sustained atrial arrhythmias can be reduced.

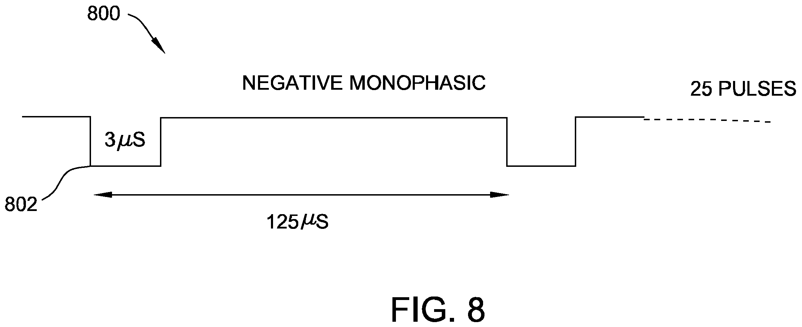

Figure 8 is an embodiment of a window 800 that is part of a larger monophasic waveform. Window 800 includes a series of monophasic pulses 802. For example, in this embodiment, window 800 includes twenty five monophasic pulses 802, with a pulse period (i.e., the time between the beginning of one pulse 802 and the beginning of a subsequent pulse 802) of approximately 125µs. Further, each pulse 802 is a negative pulse having a pulse width of approximately 3µs. Those of skill in the art will appreciate that the values are examples, and that any suitable number of pulses, pulse period, pulse polarity, and/or pulse width may be used.

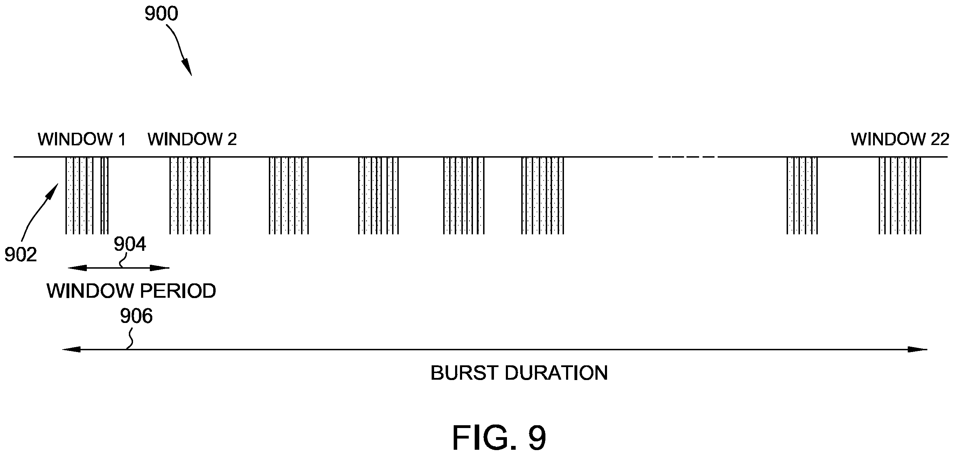

Figure 9 is an embodiment of a waveform 900 including a plurality of windows 902, such as window 800 (shown in Figure 8). Each window 800 includes a plurality of pulses such as monophasic pulses 802 (shown in Figure 8). In this embodiment, waveform 900 includes twenty two windows 902 (although any suitable number of windows 902 may be included in waveform 900). A window period 904 is defined as the time between the beginning of one window 902 and the beginning of a subsequent window 902. Further, a burst duration 906 is defined as the time between the beginning of the window period 904 including the first window 902 and the end of the window period 904 including the last window 902.

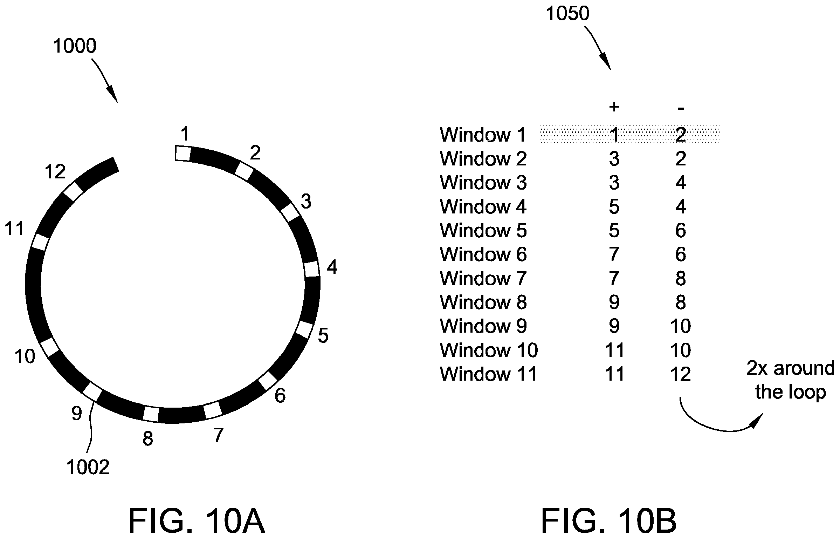

Figure 10A is a schematic diagram of a catheter assembly 1000 that may be used to deliver waveform 900. Further, Figure 10B is a chart of one embodiment of an electrode addressing scheme 1050 for waveform 900. Alternatively, waveform 900 may be implemented using any suitable catheter or electrode addressing scheme.

Catheter assembly 1000 is a loop catheter, similar to catheter assembly 146 (shown in Figures 2A and 2B). In this embodiment, catheter assembly 1000 includes twelve electrodes 1002, with each electrode 1002 having a numerical designation (e.g., 1, 2, ... 12).

Electrode addressing scheme 1050 specifies which electrodes 1002 are used to generate the pulses within each window 902 of waveform 900. In this embodiment, pulses are generated between pairs of adjacent electrodes 1002, with a different pair used for each window 902, the pairs moving sequentially around the loop of catheter assembly 1000. This results in eleven windows 902, and the pattern is repeated twice to arrive at twenty two windows 902.

In one embodiment, window period 904 is approximately 8ms and burst duration 906 is approximately 176ms. Each window 902 may include, for example, twenty five pulses with a pulse period of approximately 125µs and a pulse width of approximately 3µs (similar to window 800 shown in Figure 8).

As noted above, however, reducing burst duration 906 may reduce the likelihood of generation of sustained atrial arrhythmias. Accordingly, in another embodiment, window period 904 is less than approximately 8ms, and burst duration 906 is less than approximately 176ms. For example, window period 904 may be less than 7ms, more particularly may be less than 5ms, and even more particularly, may be approximately 3.5ms. Further, burst duration 906 may be less than 150ms, more particularly may be less than 100ms, and even more particularly may be approximately 78ms. A burst duration 906 of approximately 78ms is less than half of a burst duration 906 of approximately 176ms.

In one example of a waveform with a reduced window period 904 and a reduced burst duration 906, the pulse width is approximately 3µs and the pulse period is approximately 113µs. Alternatively, any suitable pulse width and pulse period may be used.

Reducing the burst duration has been shown to significantly reduce the likelihood of arrhythmia. In one example, arrhythmia levels were compared for a first waveform having a burst duration of 176ms and a second waveform having a burst duration of 78ms.

For the first waveform (having a burst duration of 176ms), several trials were conducted. 46% of the total trials had no arrhythmia, 54% of the total trials had arrhythmia, and 35% of the total trials had sustained atrial arrhythmia/flutter that required pacing to terminate.

For the second waveform (having a burst duration of 78ms), several trials were also conducted. Notably, for the second waveform, 97.5% of the trials had no arrhythmia, and only 2.5% of the total trials had arrhythmia. The trials with arrhythmia all had sustained atrial arrhythmia/flutter that terminated on its own without requiring pacing (i.e., 0% of the total trials had sustained atrial arrhythmia/flutter that required pacing to terminate). Accordingly, the arrhythmia level was significantly reduced (Pearson χ2: p < 0.001) for the shorter burst duration waveform.

Further, experimental results indicated that lesion surface areas and lesion depth using the shorter burst duration waveform were not significantly different from lesion surface areas and lesion depth using the longer burst duration waveform. In addition, experimental results indicated that neither waveform resulted in thermal damage to ablated tissue. However, the shorter burst duration waveform reduced therapy time (while maintaining the same number of bursts) and reduced the likelihood of arrhythmia.

The systems and methods described herein are directed to electroporation. An electroporation system includes a catheter including a plurality of electrodes, and a pulse generator coupled to the catheter, the pulse generator configured to generate a waveform to be delivered using at least one of the plurality of electrodes. The waveform includes a first pulse having a first polarity, a first pulse amplitude, and a first pulse width, and a second pulse having a second polarity, a second pulse amplitude, and a second pulse width, wherein the first and second pulses are separated by an interpulse delay, and wherein at least one of i) the first pulse amplitude is different than the second pulse amplitude and ii) the first pulse width is different than the second pulse width.

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense.

## Claims

1. An electroporation system comprising:
a catheter (14) comprising a plurality of electrodes; and
a pulse generator (26) coupled to the catheter (14), the pulse generator (26) configured to generate a waveform to be delivered using at least one of the plurality of electrodes, the waveform including:
a plurality of windows (902), each window including a plurality of pulses (802), wherein the waveform has a burst duration (906) that is less than approximately 176 milliseconds (ms) and a window period (904) that is less than approximately 8ms.

2. The electroporation system in accordance with claim 1, wherein the burst duration (906) is less than 150ms.

3. The electroporation system in accordance with claim 1, wherein the burst duration (906) is less than 100ms.

4. The electroporation system in accordance with claim 1, wherein the burst duration (906) is approximately 78ms.

5. The electroporation system in accordance with any one of claims 1-4, wherein the window period (904) is less than 7ms.

6. The electroporation system in accordance with any one of claims 1-4, wherein the window period (904) is less than 5ms.

7. The electroporation system in accordance with any one of claims 1-4, wherein the window period (904) is approximately 3.5ms.

8. The electroporation system in accordance with any one of claims 1-7, wherein each window (902) includes at least negative pulses.

9. A pulse generator (26) for use with an electroporation system, the pulse generator (26) configured to be coupled to a catheter (14) including a plurality of electrodes and configured to generate a waveform to be delivered using at least one of the plurality of electrodes, the waveform including:
a plurality of windows (902), each window including a plurality of pulses,
wherein the waveform has a burst duration (906) that is less than approximately 176 milliseconds (ms) and a window period (904) that is less than approximately 8ms.

10. The pulse generator in accordance with claim 9, wherein the burst duration (906) is less than 150ms.

11. The pulse generator in accordance with claim 9, wherein the burst duration (906) is less than 100ms.

12. The pulse generator in accordance with claim 9, wherein the burst duration (906) is approximately 78ms.

13. The pulse generator in accordance with any one of claims 9-12, wherein the window period (904) is less than 7ms.

14. The pulse generator in accordance with any one of claims 9-12, wherein the window period (904) is less than 5ms.

15. The pulse generator in accordance with any one of claims 9-12, wherein the window period (904) is approximately 3.5ms.
